# EUROPÄISCHE PATENTSCHRIFT

(11) **EP 0 294 560 B1**
(45) Veröffentlichungstag und Bekanntmachung des Hinweises auf die Patenterteilung: **28.07.1993**
(21) Anmeldenummer: 88105863.0
(22) Anmeldetag: 13.04.1988
(51) Int. Cl.: C07C 63/16, C07C 51/265, C07C 51/31

(54) **Verfahren zur Herstellung von Phthalsäureanhydrid durch Gasphasenoxidation eines Orthoxylol-Naphthalin-Gemisches**
Process for preparing phtalic anhydride by a vapour-phase oxidation of an ortho-xylene-naphthalene mixture
Procédé de préparation d'anhydride phtalique par oxydation en phase gazeuse d'un mélange d'ortho-xylène-naphtalène

(30) Priorität: 11.06.1987 DE 3719476
(43) Veröffentlichungstag der Anmeldung: 14.12.1988
(73) Patentinhaber: HÜLS AKTIENGESELLSCHAFT, 45764 Marl (DE)
(72) Erfinder: Fuhrmann, Werner, Dr., D-4358 Haltern (DE); zur Hausen, Manfred, Dr., D-4370 Marl (DE); Krix, Wilfried, D-4250 Bottrop (DE)

(56) Entgegenhaltungen:
- DE-C- 1 793 453

## Beschreibung

Die Gasphasenoxidation von o-Xylol und/oder Naphthalin zu Phthalsäureanhydrid ist bekannt und in der Literatur vielfach belegt. Auch die Oxidation von o-Xylol-Naphthalin-Gemischen ist beschrieben. So wird in der BE-PS 893 521 der o-Xylol-Einsatz mit zunehmendem Alter des Katalysators durch Naphthalin ergänzt, Produktions- oder Qualitätseinbußen müssen dadurch nicht hingenommen werden. Es ist auch bekannt, daß die Oxidation von o-Xylol-Naphthalin-Gemischen deutlich höhere Ausbeuten an Phthalsäureanhydrid liefert als die Umsetzung der einzelnen Kohlenwasserstoffe allein (J. Appl. Chem. USSR 41 (1968), Seiten 2 223 und 2 224). Diese Tatsache sowie andere Vorteile machen die Mischoxidation zu einem technisch besonders interessanten Verfahren.

Großtechnisch wird Phthalsäureanhydrid aus o-Xylol und/oder Naphthalin hergestellt. Bei der Gasphasenoxidation des o-Xylols wird der vorgeheizte Kohlenwasserstoff in ca. 170 °C heiße Prozeßluft eingedüst und dieses o-Xylol-Luft-Gemisch der Oxidation zugeführt. Dagegen bedienen sich die Verfahren zur Oxidation des Naphthalins des sogenannten Verdampferprinzips. Hierbei wird ein Primärluftstrom durch Einleiten in eine ca. 140 °C heiße Naphthalinschmelze mit Kohlenwasserstoff beladen. Dieses Naphthalin-Luft-Gemisch wird nach Verdünnung mit einer weiteren Sekundärluftmenge der katalytischen Oxidation unterworfen. Für die beiden Prozesse folgt hieraus in Abhängigkeit von der Art des Rohstoffs eine individuell unterschiedliche verfahrenstechnische Auslegung (H. Suter Phthalsäureanhydrid und seine Verwendung, Darmstadt 1972, Seite 51). Ohne ergänzende Maßnahmen ist daher eine zur Oxidation von o-Xylol konzipierte Anlage für den Einsatz von Naphthalin nicht geeignet und umgekehrt.

Verfahren zur Gasphasenoxidation von o-Xylol-Naphthalin-Gemischen unter Wahrung des Verdampferprinzips für Naphthalin einerseits und der Eindüsung des o-Xylols andererseits sind bekannt. Die Kohlenwasserstoff-Luft-Gemische werden in getrennten, parallelen Strängen nach den für sie eigenen Verfahrensprinzipien erzeugt, vereinigt und gemeinsam dem Reaktor zur katalytischen Gasphasenoxidation aufgegeben. Diese Verfahrensweise zur Mischoxidation hat sich entwickelt aus dem Prozeß der Naphthalinoxidation unter Ergänzung derjenigen Apparateteile, die eine zusätzliche Verdüsung von o-Xylol ermöglichen.

Die Flexibilität gegenüber den Rohstoffen stellt für die Bewertung eines Verfahrens ein wesentliches Kriterium dar (H. Suter, Phthalsäureanhydrid und seine Verwendung, Darmstadt 1972, Seite 62). Ein Prozeß, der unter möglichst identischen Verfahrensbedingungen o-Xylol und Naphthalin verarbeiten kann und betriebssicher ist, bleibt das erklärte Ziel einer solchen verfahrenstechnischen Entwicklung. Der Naphthalineinsatz im Gemisch mit o-Xylol unter Verzicht des Verdampferprinzips entspricht dieser Vorstellung auch unter sicherheitstechnischen Aspekten, denn der Verdampfer ist aufgrund seines Volumens und der enthaltenen Produktmenge vor allem beim Low-Energy-Process mit besonderen Risiken behaftet.

Die Möglichkeit der Eindüsung des Naphthalins entsprechend dem o-Xylol-Prozeß ist in der Literatur erwähnt (H. Suter, Phthalsäureanhydrid und seine Verwendung, Darmstadt 1972, Seite 49), ein Verfahren auf Basis dieser Technik steht jedoch aus. Ursache hierfür sind eine Reihe von Schwierigkeiten bei der Verfahrensdurchführung, bedingt durch die besonderen chemischen und physikalischen Eigenschaften des Naphthalins. Die Schwierigkeiten treten besonders dann auf, wenn das bei der Verfahrensdurchführung in heiße Prozeßluft eingespritzte Kohlenwasserstoff-Gemisch mehr als 20 Gewichtsteile Naphthalin enthält. Bei höheren Naphthalinanteilen führt die Mischoxidation zur Bildung organischer Ablagerungen im Katalysatorbett, diese bewirken innerhalb kürzester Zeit Differenzdruckanstiege am Reaktor, verbunden mit der Notwendigkeit einer Zurücknahme der Naphthalineinspeisung bzw. Abstellung der Reaktion.

Der vorliegenden Erfindung liegt die Aufgabe zugrunde, diese Nachteile des Verfahrens zu beseitigen.

Gegenstand der Erfindung ist ein Verfahren zur Herstellung von Phthalsäureanhydrid aus einem Gemisch von o-Xylol und Naphthalin durch Gasphasenuoxidation an Metalloxid-Katalysatoren, welches dadurch gekennzeichnet ist, daß man
- eine bei 0 bis 80 °C gelagerte Lösung von 1 bis 80 Gewichtsteilen Naphthalin in 99 bis 20 Gewichtsteilen o-Xylol oder
- frisch destilliertes, innerhalb einer Verweilzeit bis 60 Minuten bei 80 bis 120 °C gehaltenes Naphthalin in Lösung mit o-Xylol
auf 110 bis 180 °C erhitzt, die erhitzte Lösung in einem heißen Luftstrom von 150 bis 200 °C eindüst und über Metalloxid-Katalysatoren leitet.

Zur Herstellung von Phthalsäureanhydrid durch Gasphasenoxidation setzt man erfindungsgemäß eine Lösung von Naphthalin in o-Xylol ein, wobei diese Lösung vorzugsweise bei Temperaturen unterhalb des Erstarrungspunktes von Naphthalin gelagert und/oder Verweilzeiten oberhalb 78 °C nur kurz bemessen sind. Als Naphthalinqualitäten kommen bevorzugt destilliertes, insbesondere frisch destilliertes Naphthalin und/oder Naphthalinkristallisate, insbesondere solche vom Erstarrungspunkt 78 bis 80 °C, in Betracht. Geeignet sind auch andere Naphthalinqualitäten.

Bei dem erfindungsgemäßen Verfahren stellt man zunächst bei einer Temperatur von 0 bis 80 °C, vorzugsweise 30 bis 70 °C, insbesondere 40 bis 60 °C, eine Lösung des Naphthalins, vorzugsweise des in der Kristallisation und/oder Destillation anfallenden Naphthalins, in o-Xylol her. Anschließend lagert man die o-Xylol-Naphthalin-Lösung bei 0 bis 80 °C, vorzugsweise bei 30 bis 70 °C, insbesondere bei 40 bis 60 °C. Auf diese Weise sind Lösungen von 1 bis 80 Gewichtsteilen Naphthalin, bevorzugt von 30 bis 65 Gewichtsteilen, insbesondere von 40 bis 60 Gewichtsteilen Naphthalin, in 99 bis 20 Gewichtsteilen, vorzugsweise von 70 bis 35 Gewichtsteilen, insbesondere von 60 bis 40 Gewichtsteilen, o-Xylol herstellbar und bei diesen Temperaturen praktisch zeitlich unbegrenzt, vorzugsweise bis zu 10 Tagen, lagerfähig, wobei man für die Naphthalin-reichen Lösungen die höheren Temperaturen und für die Naphthalin-armen Lösungen vorzugsweise die tieferen Temperaturen wählt. Die bei der Lagerung gegebenenfalls ausgefallenen unlöslichen Anteile werden vorzugsweise abgetrennt.

Die Lösung von Naphthalin in o-Xylol wird kurz vor der Reaktion aus dem Vorratsbehälter abgepumpt, auf Temperaturen von 110 bis 180 °C erhitzt, die erhitzte Lösung in einen heißen Luftstrom von 150 bis 200 °C eingedüst und in bekannter Weise über Metalloxid-Katalysatoren geleitet. Bevorzugt setzt man TiO₂-V₂O₅-Katalysatoren, insbesondere dotierte TiO₂-V₂O₅-Katalysatoren ein.

In einer speziellen Ausführung des erfindungsgemaßen Verfahrens wird eine o-Xylol-Naphthalin-Lösung in höherer Konzentration, bis 80 Gewichtsteilen Naphthalin, hergestellt. Diese Lösung, dessen Temperatur zunächst weniger als 80 °C beträgt, wird aus dem Vorratsbehälter abgepumpt, auf Temperaturen von 110 bis 180 °C erhitzt und mit weiteren Mengen heißen o-Xylols bis auf Konzentrationen von 1 bis < 80 Gewichtsteilen Naphthalin verdünnt, bevor diese Lösung in heiße Prozeßluft eingedüst und der Gasphasenoxidation zugeführt wird. Alternativ kann das Aufheizen auf 110 bis 180 °C auch erst nach Abmischen mit den zusätzlichen Mengen o-Xylol erfolgen.

Bei einer bevorzugten Ausführung des erfindungsgemäßen Verfahrens wird Naphthalin destilliert, das frische Destillat aus der Vorlage, vorzugsweise innerhalb einer Verweilzeit bis 60 Min, insbesondere bis 20 Minuten, beispielsweise von 15 bis 45 Minuten, und einer Temperatur von 80 bis 120 °C abgepumpt, mit 99 bis 20 Gewichtsteilen o-Xylol zu einer Lösung, bestehend aus 1 bis 80 Gewichtsteilen Naphthalin, vermischt, die o-Xylol-Naphthalin-Lösung auf 110 bis 180 °C erhitzt, die erhitzte Lösung in heiße Prozeßluft von 150 bis 200 °C eingedüst und in bekannter Weise über Metalloxid-Katalysatoren geleitet.

Das erfindungsgemäße Verfahren erlaubt die störungsfreie Oxidation eines o-Xylol-Naphthalin-Gemisches durch direkte Eindüsung der Kohlenwasserstoffe in heiße Prozeßluft. Unter den milden Bedingungen dieses Verfahrens ist es gelungen, die früheren Schwierigkeiten dieses Prozesses zu überwinden. - Überraschend wurde zusätzlich gefunden, daß der Naphthochinongehalt im Roh-PSA der Mischoxidation nur 1/10 des Wertes bei reiner Naphthalinfahrweise beträgt.

Die folgenden Beispiele dienen der weiteren Erläuterung:

### Beispiel 1

Naphthalin-Destillat vom Erstarrungspunkt 79 °C wird der Destillationsvorlage entnommen und mit vorgelegtem, kalten o-Xylol vermischt, so daß eine Lösung der Kohlenwasserstoffe mit 60 Gewichtsteilen Naphthalin und einer Temperatur von 60 °C entsteht. Das o-Xylol-Naphthalin-Gemisch wird unter diesen Bedingungen 20 Tage gelagert. Danach wird es aus dem Vorratsbehälter abgepumpt, mit weiteren Mengen heißen o-Xylols von 160 °C vermischt, so daß die resultierende Mischung 50 Gewichtsteile Naphthalin enthält, diese auf eine Temperatur von 160 °C erhitzt, die erhitzte Lösung in heiße Prozeßluft von 170 °C eingedüst und über TiO₂/V₂O₅-Katalysatoren eines 12.000-Rohr-Reaktors geleitet.

Die Beladung beträgt 60 g Kohlenwasserstoffe pro Nm³ Luft bei 3,5 Nm³ pro Rohr und Stunde, die Salzbadtemperatur ist auf 371 °C eingestellt. Die Mischoxidation läuft glatt und problemlos ab, ohne daß sich während der Versuchsdauer von z. B. 25 Tagen irgendwelche Differenzdruckanstiege bemerkbar machen.

### Beispiel 2

Naphthalin wird destilliert, daß Destillat (Erstarrungspunkt 79 °C) aus der Vorlage mit einer Verweilzeit von 20 Min. und einer Temperatur von 100 °C abgepumpt und mit einem heißen o-Xylol-Strom von 165 °C so vermischt, daß eine Lösung der Kohlenwasserstoffe mit 50 Gewichtsteilen Naphthalin entsteht; dieses Gemisch wird auf eine Temperatur von 142 °C erhitzt und unter den Bedingungen des Beispiels 1 an TiO₂/V₂O₅-Katalysatoren oxidiert. Die Mischoxidation läuft glatt und problemlos ab, ohne daß sich Differenzdruckanstiege bemerkbar machen.

### Vergleichsbeispiel 3

Naphthalin-Destillat vom Erstarrungspunkt 79 °C wird bei einer Temperatur von 100 bis 110 °C gelagert. Die Verweilzeit unter diesen Bedingungen beträgt 20 Tage. Danach wird das Produkt aus dem Vorratsbehälter abgepumpt, mit heißem o-Xylol von 160 °C zu einem Kohlenwasserstoff-Gemisch mit 50 Gewichtsteilen Naphthalin vermischt, die Lösung der Kohlenwasserstoffe auf eine Temperatur von 160 ° erhitzt, die erhitzte Lösung wie in den Beispielen 1 und 2 in heiße Prozeßluft von 170 °C eingedüst und über TiO₂/V₂O₅-Katalysatoren eines 12.000-Rohr-Reaktors geleitet.

Die Beladung beträgt wiederum 60 g Kohlenwasserstoffe pro Nm³ Luft bei 3,5 Nm³ pro Rohr und Stunde, die Salzbadtemperatur ist ebenfalls auf 371 °C eingestellt.

Der Differenzdruck über dem Katalysator beträgt zu Beginn des Versuchs 335 mbar, mit dem Betrieb der Mischoxidation steigt er allmählich und kontinuierlich an, erreicht nach 3 Tagen 352 mbar, um anschließend sehr schnell auf über 400 mbar anzusteigen. Nur durch Zurücknahme des Naphthalinanteils im Kohlenwasserstoff-Gemisch auf 20 Gewichtsteile ist eine Stabilisierung des Differenzdruckes möglich. Ein geringfügiges Absenken erhöhter Werte ist zwar durch vollständige Wegnahme der Naphthalin-Einspeisung möglich, eine Regeneration des Differenzdruckes jedoch nur nach Abstellung der Reaktion.

## Patentansprüche

1. Verfahren zur Herstellung von Phthalsäureanhydrid aus einem Gemisch von o-Xylol und Naphthalin durch Gasphasenoxidation an Metalloxid-Katalysatoren,
dadurch gekennzeichnet,
daß man
- eine bei 0 bis 80 °C gelagerte Lösung von 1 bis 80 Gewichtsteilen Naphthalin in 99 bis 20 Gewichtsteilen o-Xylol oder
- frisch destilliertes, innerhalb einer Verweilzeit bis 60 Minuten bei 80 bis 120 °C gehaltenes Naphthalin in Lösung mit o-Xylol
auf 110 bis 180 °C erhitzt, die erhitzte Lösung in einem heißen Luftstrom von 150 bis 200 °C eindüst und über Metalloxid-Katalysatoren leitet.

2. Verfahren nach Anspruch 1,
dadurch gekennzeichnet,
daß man Lösungen aus 30 bis 65 Gewichtsteilen, vorzugsweise 40 bis 60 Gewichtsteilen Naphthalin und 70 bis 35, vorzugsweise 60 bis 40 Gewichtsteilen o-Xylol, herstellt.

3. Verfahren nach den Ansprüchen 1 und 2,
dadurch gekennzeichnet,
daß man zur Herstellung der Lösung ein Naphthalin-Kristallisat, vorzugsweise vom Erstarrungspunkt 78 bis 80 °C, einsetzt.

4. Verfahren nach einem der Ansprüche 1 bis 3,
dadurch gekennzeichnet,
daß man die Lösung bei einer Temperatur von 30 bis 70 °C, vorzugsweise von 40 bis 60 °C, herstellt.

5. Verfahren nach einem der Ansprüche 1 bis 4,
dadurch gekennzeichnet,
daß man die erhitzte Lösung mit einer höheren Konzentration, bis 80 Gewichtsteilen Naphthalin, mit weiteren Mengen o-Xylol bis auf Konzentrationen von 1 bis < 80 Gewichtsteilen Naphthalin verdünnt und dann in einen heißen Luftstrom von 150 bis 200 °C eindüst.

6. Verfahren nach einem der Ansprüche 1 bis 4,
dadurch gekennzeichnet,
daß man die Lösung mit einer höheren Konzentration, bis 80 Gewichtsteilen Naphthalin, vor dem Erhitzen mit weiteren Mengen o-Xylol bis auf Konzentrationen von 1 bis < 80 Gewichtsteilen Naphthalin verdünnt und dann in einen heißen Luftstrom von 150 bis 200 °C eindüst.

## Claims

1. A method for the manufacture of phthalic anhydride from a mixture of o-xylene and naphthalene by gas phase oxidation on metal oxide catalysts, characterized in that
- a solution, stored at 0 to 80°C, of 1 to 80 parts by weight naphthalene in 99 to 20 parts by weight o-xylene, or
- freshly distilled naphthalene held at 80 to 120°C, with a storage time of up to 60 minutes, in solution in o-xylene
is heated to 110 to 180°C, the heated solution is sprayed into a hot air stream having a temperature of 150 to 200°C and is led over metal oxide catalysts.

2. A method according to claim 1, characterized in that solutions of 30 to 65 parts by weight, preferably 40 to 60 parts by weight, of naphthalene and 70 to 35 parts by weight, preferably 60 to 40 parts by weight, of o-xylene are prepared.

3. A method according to claims 1 and 2, characterized in that a naphthalene crystallate preferably with a melting point of 78 to 80°C, is used to prepare the solution.

4. A method according to any one of claims 1 to 3, characterized in that the solution is prepared at a temperature of 30 to 70°C, and preferably at 40 to 60°C.

5. A method according to any one of claims 1 to 4, characterized in that the heated solution containing a high concentration, up to 80 parts by weight, of naphthalene is diluted with further amounts of o-xylene to a concentration of 1 to < 80 parts by weight of naphthalene and is then sprayed into a hot air stream having a temperature of 150 to 200°C.

6. A method according to any one of claims 1 to 4, characterized in that the solution containing a high concentration, up to 80 parts by weight, of naphthalene is diluted before heating with further amounts of o-xylene to a concentration of 1 to < 80 parts by weight of naphthalene and is then sprayed into a hot air stream having a temperature of 150 to 200°C.

## Revendications

1. Procédé de production d'anhydride phtalique à partir d'un mélange d'o-xylène et de naphtalène par oxydation en phase gazeuse sur des catalyseurs à base d'oxyde métallique, caractérisé en ce que l'on chauffe de 100 à 180°C :
- soit une solution conservée entre 0 et 80°C de 1 à 80 parties en poids de naphtalène dons 99 à 20 parties en poids d'o-xylène,
- soit du naphtalène fraîchement distillé, maintenu entre 80 et 120°C pendant un temps de séjour allant jusqu'à 60 min en solution avec de l'o-xylène,
et que l'on introduit la solution chauffée, dans un courant d'air chaud à une température de 150 à 200°C et qu'on la dirige sur des catalyseurs à base d'oxyde métallique.

2. Procédé selon la revendication 1, caractérisé en ce que l'on prépare des solutions à base de 30 à 65 parties en poids - de préférence de 40 à 60 parties en poids - de naphtalène et de 70 à 35 - de préférence de 60 à 40 - parties en poids d'o-xylène.

3. Procédé selon les revendications 1 et 2, caractérisé en se que l'on utilise pour la production de la solution une forme cristalline de naphtalène - de préférence celle ayant un point de solidification de 78 à 80°C.

4. Procédé selon l'une des revendications 1 à 3, caractérisé en ce que l'on prépare la solution à une température allant de 30 à 70°C - de préférence de 40 à 60°C.

5. Procédé selon l'une des revendications 1 à 4, caractérisé en ce que l'on dilue la solution chauffée ayant une concentration plus élevée allant jusqu'à 80 parties en poids de naphtalène avec des quantités supplémentaires d'o-xylène jusqu'à des concentrations allant de 1 à < 80 parties en poids de naphtalène et ensuite qu'on l'injecte dans un courant d'air chaud de 150 à 200°C.

6. Procédé selon l'une des revendications 1 à 4, caractérisé en ce que l'on dilue la solution ayant une concentration plus élevée jusqu'à 80 parties en poids de naphtalène, avant le chauffage avec des quantités supplémentaires d'o-xylène jusqu'à des concentrations allant de 1 à < 80 parties en poids de naphtalène et qu'ensuite on l'injecte dans un courant d'air chaud de 150 à 200°C.
